# EUROPÄISCHE PATENTSCHRIFT

(11) **EP 0 677 509 B1**
(45) Veröffentlichungstag und Bekanntmachung des Hinweises auf die Patenterteilung: **14.01.1998**
(21) Anmeldenummer: 94111215.3
(22) Anmeldetag: 19.07.1994
(51) Int. Cl.: C07C 231/12, C07C 233/36, C11D 1/90

(54) **Verfahren zur Herstellung hochkonzentrierter fliessfähiger wässriger Lösungen von Betainen**
Process for the preparation of highly concentrated, freely flowable, aqueous solutions of betaines
Procédé pour la préparation de dispersions aqueuses de bétaines fortement concentrées et fluides

(30) Priorität: 12.04.1994 DE 4412481
(43) Veröffentlichungstag der Anmeldung: 18.10.1995
(73) Patentinhaber: Witco Surfactants GmbH, D-36396 Steinau an der Strasse (DE)
(72) Erfinder: Hamann, Ingo, Dr., D-63619 Bad Orb (DE); Köhle, Hans-Jürgen, Dr., D-36381 Schlüchtern (DE); Wehner, Winfried, Dr., D-36119 Neuhof (DE)

(56) Entgegenhaltungen:
- EP-A- 0 077 674
- EP-A- 0 353 580
- EP-A- 0 557 835
- DE-C- 3 726 322
- DE-C- 4 207 386
- US-A- 3 225 074

## Beschreibung

Die Erfindung betrifft ein Verfahren zur Herstellung hochkonzentrierter fließfähiger wäßriger Lösungen von Betainen, welche Feststoffgehalte von bis zu 50 Gew.-% enthalten.

Betaine haben sich in den letzten Jahren in der kosmetischen Industrie als fester Bestandteil von Rezepturen, insbesondere für die Haar- und Körperreinigung, etabliert. Sie haben die Fähigkeit, einen dichten und sahnigen Schaum auszubilden, welcher auch in Gegenwart anderer Tenside, Seifen und Additive über einen langen Zeitraum stabil bleibt, verbunden mit anerkannt guten Reinigungseigenschaften ohne irgendwelche irritierenden Nebenwirkungen, selbst für empfindliche Haut.

Die Herstellung von Betainen wird in der einschlägigen Patent- und Fachliteratur ausführlich beschrieben (US-PS 3 225 074). Im allgemeinen werden dabei tertiäre Aminstickstoffatome enthaltende Verbindungen mit ω-Halogencarbonsäuren oder deren Salze in wäßrigen oder wasserhaltigen Medien umgesetzt. Als tertiäre Aminstickstoffatome enthaltende Verbindungen werden insbesondere Fettsäureamide der allgemeinen Formel (I)

R³-CONH-(CH₂)ₘ-NR⁴R⁵ (I)

eingesetzt, worin R³ der Alkylrest einer Fettsäure, welcher gegebenenfalls verzweigt, gegebenenfalls Mehrfachbindungen, gegebenenfalls Hydroxylgruppen enthalten kann, R⁴ und R⁵ gleich oder verschiedenen Alkylreste mit 1 - 4 C-Atomen bedeuten und m = 1 - 3 sein kann.

Der Alkylrest R³ kann sich hierbei von den natürlichen oder synthetischen Fettsäuren mit 6 - 20 C-Atomen, vorzugsweise von den natürlichen pflanzlichen oder tierischen Fettsäuren mit 8 - 18 C-Atomen ableiten sowie deren natürlich vorkommenden speziell eingestellten Mischungen miteinander oder untereinander.

Als Fettsäuren kommen beispielsweise Capronsäure, Caprylsäure, Caprinsäure, Laurinsäure, Palmitinsäure, Stearinsäure, Linolsäure, Linolensäure, Ricinolsäure in Betracht.

Bevorzugt werden die natürlich vorkommenden Fettsäuremischungen mit einer Kettenlänge von 8 - 18 C-Atomen, wie Kokosfettsäure oder Palmkernfettsäure, welche gegebenenfalls durch geeignete Hydrierungsmethoden gehärtet werden können.

Mit diesen Fettsäuren bzw. Fettsäuremischungen wird durch übliche Kondensationsreaktion bei 140 - 200 °C mit Aminen der allgemeinen Formel (II)

H₂N-(CH₂)ₘ-NR⁴R⁵ (II)

- in welcher R⁴ und R⁵ und m die in der Formel (I) genannte Bedeutung haben - zu den Fettsäureamiden mit tertiären Stickstoffatomen der allgemeinen Formel (I) umgesetzt.

Die anschließende Quaternierungsreaktion zu Betainen der Formel (III)

R³-CONH-(CH₂)ₘ-N⁺R⁴R⁵(CH₂)_{y}COO⁻ (III)

worin R³, R⁴, R⁵ und m die gleiche Bedeutung wie in den Formeln (I) und (II) haben und y = 1, 2, 3 sein kann, kann nach den literaturbekannten Verfahren durchgeführt werden.

Dem Fettsäureamid der Formel (I) werden dabei in der Regel in wäßrigem Medium ω-Halogenalkylcarbonsäuren, vorzugsweise das Natriumsalz der Chloressigsäure, zugesetzt und bei einer mehrstündigen Reaktion bei ca. 80 - 100 °C die Quaternierung vollzogen. Je nach eingesetzter Fettsäure bzw. Fettsäuremischung muß zum Erhalt der Rührfähigkeit bei fortschreitender Reaktion eine Mindestmenge an Wasser vorhanden sein. Die handelsübliche Konzentration an Betaingehalt der auf diese Weise hergestellten Lösungen liegt daher bei etwa 30 Gew.-% oder darunter.

Zur Einsparung von Lagerungs- und Transportkosten sowie aus formulierungstechnischen Gründen bei der Weiterverarbeitung war aber in vielen Fällen eine höhere Konzentration dringend erwünscht.

In der Vergangenheit sind daher eine Reihe von Verfahren vorgeschlagen worden, welche dieses Problem lösen sollten. So ist aus der DE-PS 3 613 944 ein Verfahren bekannt, bei dem die Quaternierung in einem organischen polaren Lösungsmittel mit einem Wasseranteil von 20 Gew.-% durchgeführt und danach das Lösungsmittel ganz oder teilweise destillativ entfernt und dann wieder mit einem anwendungstechnisch brauchbaren Lösungsmittel auf die gewünschte Konzentration eingestellt wird.

Abgesehen davon, daß das Verfahren technisch aufwendig und kostenintensiv ist, sind organische Lösungsmittel bei der Weiterverarbeitung in kosmetischen Rezepturen vielfach unerwünscht.

Das Verfahren gemäß DE-PS 3 726 322 kommt zwar ohne organische Lösungsmittel aus, jedoch muß die für die Quaternierungsreaktion erforderliche Wassermenge durch Destillation wieder aus dem Reaktionsprodukt entfernt werden, wobei vor oder nach der Einstellung auf die gewünschte Konzentration der pH-Wert der Lösung durch relativ hohe Säuremengen auf hautuntypische Werte von 1 - 4,5 eingestellt werden muß.

Gemäß EP-A-0 353 580 werden dem Reaktionsgemisch aus Fettsäureamid und Halogenalkylcarbonsäure vor oder während der Quaternierungsreaktion oder der erhaltenen Lösung des Betains nichtionogene, wasserlösliche Tenside in solchen Mengen zugesetzt, daß die fertige Lösung 3 - 20 Gew.-% wasserlösliche Tenside enthält.

Als nichtionogene Tenside werden Polyoxyethylenether eingesetzt, welche für eine ausreichende Wasserlöslichkeit 10 - 250 Oxyethyleneinheiten enthalten müssen.

Polyoxyethylenether mit höheren Anteilen an Oxyethyleneinheiten haben sich hinsichtlich ihrer biologischen Abbaubarkeit aber als nicht unproblematisch erwiesen.

Es bestand daher weiterhin ein Bedarf an hochkonzentrierten, fließ- und pumpfähigen, wäßrigen Lösungen von Betainen, welche frei von niedrigen Alkoholen wie Methanol, Ethanol, Propanol oder Isopropanol sind.

Überraschenderweise wurde nun gefunden, daß man fließ- und pumpfähige bis zu ca. 45 Gew.-prozentige Lösungen von Betainen, bezogen auf Trockensubstanz, aus dem Reaktionsgemisch aus Fettsäureamid und ω-Halogenalkylcarbonsäure nach bekannten Verfahren herstellen kann, wenn man während der Quaternierungsreaktion Erdalkalihydroxyde wie insbesondere Mg(OH)₂ und(oder Ca(OH)₂ zusetzt.

Gegenstand der Erfindung ist daher ein Verfahren zur Herstellung hochkonzentrierter fließ- und pumpfähiger wäßriger Lösungen von Betainen der allgemeinen Formel (III)

R³-CONH-(CH₂)ₘ-N⁺R⁴R⁵(CH₂)_{y}COO⁻ (III)

worin R³ der Alkylrest einer Fettsäure, welcher gegebenenfalls verzweigt, gegebenenfalls Mehrfachbindungen, gegebenenfalls Hydroxylgruppen enthalten kann, R⁴ und R⁵ gleich oder verschiedenen Alkylreste mit 1 - 4 C-Atomen bedeuten und m = 1 - 3 und y = 1, 2, 3 sein kann, durch Quaternierung von tertiären Aminstickstoff enthaltenden Verbindungen mit ω-Halogencarbonsäuren nach bekannten Verfahren, welches dadurch gekennzeichnet ist, daß der Reaktionsmischung vor oder während der Quaternierungsreaktion a) 30 - 100 Equivalent-%, bezogen auf eingesetzte ω-Halogencarbonsäure, Mg(OH)₂ und/oder Ca(OH)₂ und b) 0 - 70 Equivalent-%, bezogen auf eingesetzte ω-Halogencarbonsäure, NaOH, KOH, NH₄OH, zugesetzt werden.

Ein weiterer Gegenstand der Erfindung betrifft die Abänderung dieses Verfahrens, welches dadurch gekennzeichnet ist, daß als Komponente a) 30 - 100 Equivalent-%, bezogen auf eingesetzte ω-Halogencarbonsäure, LiOH eingesetzt wird.

Bei der Durchführung des erfindungsgemäßen Verfahrens geht man so vor, daß die vorgelegte ω-Halogencarbonsäure mit einer Mischung aus Natrium- und Magnesium- und/oder Calciumhydroxid neutralisiert und durch mehrstündige Umsetzung in wäßrigem Medium bei 80 - 100 °C die Quaternierungsreaktion der Verbindung (I) durchgeführt wird.

Eine weitere Verfahrensvariante zur Herstellung hochkonzentrierter fließ- und pumpfähiger wäßriger Lösungen von Betainen besteht darin, daß die Reaktion nach den bekannten Verfahren mit Chloressigsäure und NaOH bzw. mit dem Natriumsalz der Chloressigsäure durchgeführt wird, mit der Abänderung, daß als Viskositätsregulatoren vor oder während der Quaternierungsreaktion Lithium-, Magnesium- oder Calciumsalze allein oder in Mischung zugesetzt werden.

Ein weiterer Gegenstand der Erfindung ist daher ein Verfahren zur Herstellung hochkonzentrierter fließ- und pumpfähiger wäßriger Lösungen von Betainen durch Quaternierung von tertiären Aminstickstoff enthaltenden Verbindungen mit ω-Halogencarbonsäuren nach bekannten Verfahren, welches dadurch gekennzeichnet ist, daß man dem Fettsäureamid der allgemeinen Formel (I) und dem Na-, K- oder NH₄-Salz der ω-Halogencarbonsäure vor oder während der Quaternierungsreaktion 0,5 - 5 Gew.-%, bezogen auf Gesamtmischung, mindestens eine der Verbindungen

(Li¹⁺)ₐ(Xⁿ⁻)_{b}, (Mg²⁺)ₐ(Xⁿ⁻)_{b}, (Ca²⁺)ₐ(xⁿ⁻)_{b}

zusetzt, worin Xⁿ⁻ der Rest einer organischen oder anorganischen Säure mit n = 1 - 3 und im Molekül der Wert von a⁺ = b·n⁻ ist.

Xⁿ⁻ ist das Anion von ein- oder mehrwertigen organischen Säuren, insbesondere ein- bis dreiwertigen Säuren, soweit sie mit Li, Mg, Ca lösliche Salze bilden, wie C₁C₅-Monocarbonsäuren, vorzugsweise Essigsäure.

Erfindungsgemäß bevorzugt werden jedoch LiCl, MgCl₂, CaCl₂ in Form der wasserfreien Salze oder der Hydrate eingesetzt.

Die Mengen liegen zwischen 0,5 - 5 Gew.-%, bezogen auf Gesamtmischung.

Eine weitere Verfahrensvariante besteht darin, daß die Li⁺-, Mg²⁺-, Ca²⁺-Salze teilweise ersetzt werden durch Verbindungen der allgemeinen Formel (IV) 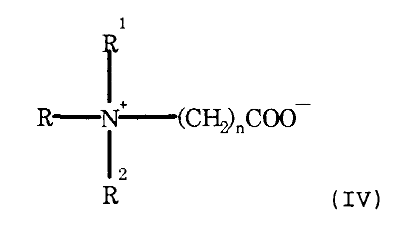 worin R, R¹, R² gleich oder verschieden gegebenenfalls verzweigter, gegebenenenfalls Hydroxylgruppen enthaltende Alkylreste mit 1 - 10 C-Atomen, insbesondere Methylreste und n = 1 - 3, vorzugsweise n = 1, ist, wobei erfindungsgemäß Mischungen aus 0,5 - 2 Gew.-% Salz und 0,5 - 5 Gew.-% Betain bevorzugt werden.

Erfindungsgemäß mitverwendete Verbindungen der allgemeinen Formel (IV) sind Quaternierungsprodukte von Dimethylethanolamin, Methyldiethanolamin oder Alkyl(C₂-C₁₀)-Dimethylamin und Monochloressigsäure, insbesondere aber das in der Zuckerrübe (Beta vulgaris) natürlich vorkommende Trimethylglycin oder "Betain".

Diese Verbindungen können als Mischungen zusammen mit den Salzen, parallel zur Salzzugabe oder nach der Salz zugabe in Mengen von 1 - 5 Gew.-%, bezogen auf die wäßrige Lösung, der Reaktionsmischung zugesetzt werden, wobei der Gesamtgehalt an Salz + Betain >5 - 7 Gew.-%, bezogen auf die wäßrige Lösung, in der Regel keine Vorteile bringt.

Erfindungsgemäß bevorzugt werden Mischungen aus 0,5 - 2 Gew.-% Salz und 0,5 - 5 Gew.-% Betain.

Die Durchführung des Verfahrens lehnt sich an die im Stand der Technik bekannten Verfahren an, wobei die wesentliche Änderung im Zusatz der Erdalkalisalze und/oder des LiCl vor oder während der Quaternierungsreaktion besteht.

Erfindungsgemäß bevorzugt wird so verfahren, daß die vorgelegte ω-Halogencarbonsäure mit Natriumhydroxid neutralisiert und vor oder während der mehrstündigen Umsetzung in wäßrigem Medium bei 80 - 100 °C 0,5 - 5,0 Gew.-%, bezogen auf die Gesamtmischung, an LiCl, MgCl₂ und/oder CaCl₂ zugegeben wird.

### Analysenmethoden

### Trockensubstanz:

Die Trockensubstanz wird durch Trocknen des Materials bei 105 °C bis zur Gewichtskonstanz bestimmt. Diese Werte werden bestimmt nach den Einheitsmethoden der Deutschen Gesellschaft für Fettchemie (DGF): B-II.

### Säurezahl (SZ):

Die Säurezahl ist ein Maß für die in Fetten und technischen Fettsäuren enthaltenen freien Säuren. Sie gibt die Anzahl Milligramm Kaliumhydroxid an, die notwendig ist, um 1 Gramm Substanz oder technische Fettsäuren zu neutralisieren (mg KOH/g). Diese Werte werden bestimmt nach den Einheitsmethoden der Deutschen Gesellschaft für Fettchemie (DGF): D-IV 2a.

### Esterzahl (EZ):

Die Esterzahl ist ein Maß für die in Fetten und technischen Fettsäuren enthaltenen Ester. Sie gibt die Anzahl Milligramm Kaliumhydroxid an, die notwendig ist, um 1 Gramm Substanz oder technische Fettsäuren zu verseifen (mg KOH/g). Diese Werte werden bestimmt nach den Einheitsmethoden der Deutschen Gesellschaft für Fettchemie (DGF): C-V 4.

### Gesamtaminzahl (GAZ), Tertiäraminzahl (TAZ):

Die Gesamtaminzahl gibt die Anzahl Milligramm Kaliumhydroxid an, die der Gesamtbasizität von 1 Gramm der Aminverbindung äquivalent sind (mg KOH/g).

Die Tertiäraminzahl gibt die Anzahl Milligramm Kaliumhydroxid an, die der Tertiäraminbasizität von 1 Gramm der Aminverbindung äquivalent sind (mg KOH/g).

Die Werte werden bestimmt nach American Oil Chemists Society (A.O.C.S.) Official Method Tf 2a-64.

### Chlorid:

Der Gehalt an Chlorid wird potentiometrisch gegen eine Silbernitrat-Maßlösung ermittelt. Als Elektrode wird eine kombinierte Silberchlorid-Elektrode verwendet. Die Werte werden bestimmt nach den Einheitsmethoden der Deutschen Gesellschaft für Fettchemie (DGF): H-III 9.

### Beispiele

### Beispiel 1:

### Herstellung des Aminamids:

98,0 kg gehärtetes Kokosöl wurden in einem Reaktor mit Rührer, Thermometer und Destillationsaufsatz unter Inertgasatmosphäre mit 56,8 kg Dimethylaminopropylamin versetzt und auf 150 - 160 °C erwärmt und unter Rückfluß gekocht. Nach Beendigung der Amidierung (Esterzahl <10 mg KOH/g) wurde der Aminüberschuß bei dieser Temperatur im Vakuum abdestilliert. Die Destillation war vollständig, als die Differenz aus Gesamtaminzahl und Tertiäraminzahl geringer als 3 mg KOH/g war. Das erhaltene Aminamid hatte eine GAZ von 170,6 mg KOH/g, eine TAZ von 168,6 mg KOH/g und eine Esterzahl von 2,8 mg KOH/g.

### Beispiel 2:

97,3 g Monochloressigsäure wurden unter Kühlung in einem 1 1 Kolben mit Rührer, Innenthermometer und pH-Elektrode mit 504 g Wasser verdünnt und mit 27,8 g Lithiumhydroxid vorsichtig neutralisiert. Nach Zusatz von 320 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 2,9 g Lithiumhydroxid benötigt. Nach ca. 9 h Reaktionszeit war die Alkylierung beendet. Man ließ die Betain-Mischung auf 50 °C abkühlen und stellte den pH-Wert mit 5,2 g Citronensäure auf 5,4 ein.

Das Endprodukt war eine klare Flüssigkeit mit einer Viskosität von 130 mPas bei 20 °C, einem Trockenrückstand von 45,7 % und einem Chlorid-Gehalt von 3,8 %.

### Beispiel 3:

97,3 g Monochloressigsäure wurden unter Kühlung in einem 1 l Kolben mit Rührer, Innenthermometer und pH-Elektrode mit 515 g Wasser verdünnt und mit 31 g Magnesiumhydroxid vorsichtig neutralisiert. Nach Zusatz von 320 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 2,9 g Magnesiumhydroxid benötigt. Nach ca. 8 h Reaktionszeit war die Alkylierung beendet. Man ließ die Betain-Mischung auf 50 °C abkühlen und stellte den pH-Wert mit 6,5 g 50 %iger Citronensäure-Lösung auf 5,1 ein.

Das Endprodukt war eine klare Flüssigkeit mit einer Viskosität von 90 mPas bei 20 °C, einem Trockenrückstand von 45,0 % und einem Chlorid-Gehalt von 3,8 %.

### Beispiel 4:

97,3 g Monochloressigsäure wurden unter Kühlung in einem 1 l Kolben mit Rührer, Innenthermometer und pH-Elektrode mit 520 g Wasser verdünnt und mit 38,9 g Calciumhydroxid vorsichtig neutralisiert. Nach Zusatz von 320 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 2,9 g Calciumhydroxid benötigt. Nach ca. 8 h Reaktionszeit war die Alkylierung beendet. Man ließ die Betain-Mischung auf 50 °C abkühlen und stellte den pH-Wert mit 3 g Citronensäure auf 5,9 ein.

Das Endprodukt war eine klare Flüssigkeit mit einer Viskosität von 170 mPas bei 20 °C, einem Trockenrückstand von 45,1 % und einem Chlorid-Gehalt von 3,6 %.

### Beispiel 5:

97,3 g Monochloressigsäure wurden unter Kühlung in einem 1 l Kolben mit Rührer, Innenthermometer und pH-Elektrode mit 500 g Wasser verdünnt und mit 7,5 g Magnesiumhydroxid und 63 g Natriumhydroxid-Lösung (50 %) vorsichtig neutralisiert. Nach Zusatz von 320 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 6,0 g Natriumhydroxid (als 50 %ige Lösung) benötigt. Nach ca. 8 h Reaktionszeit war die Alkylierung beendet. Man ließ die Betain-Mischung auf 50 °C abkühlen und stellte den pH-Wert mit 3,5 g Citronensäure auf 5,4 ein.

Das Endprodukt war eine klare Flüssigkeit mit einer Viskosität von 128 mPas bei 20 °C, einem Trockenrückstand von 44,6 % und einem Chlorid-Gehalt von 3,6 %.

### Beispiel 6:

97,3 g Monochloressigsäure wurden unter Kühlung in einem 1 l Kolben mit Rührer, Innenthermometer und pH-Elektrode mit 500 g Wasser verdünnt und mit 15 g Magnesiumhydroxid und 44 g Natriumhydroxid-Lösung (50 %) vorsichtig neutralisiert. Nach Zusatz von 320 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 4,6 g Natriumhydroxid (als 50 %ige Lösung) benötigt. Nach ca. 8 h Reaktionszeit war die Alkylierung beendet. Man ließ die Betain-Mischung auf 50 °C abkühlen und stellte den pH-Wert mit 3,5 g Citronensäure auf 5,6 ein.

Das Endprodukt war eine klare Flüssigkeit mit einer Viskosität von 210 mPas bei 20 °C, einem Trockenrückstand von 45,9 % und einem Chlorid-Gehalt von 3,7 %.

### Beispiel 7:

97,3 g Monochloressigsäure wurden unter Kühlung in einem 1 l Kolben mit Rührer, Innenthermometer und pH-Elektrode mit 500 g Wasser verdünnt und mit 82 g Natriumhydroxid-Lösung (50 %) vorsichtig neutralisiert. Nach der Neutralisation wurde die Natriumchloracetat-Mischung mit 52,4 g Magnesiumchlorid-Hexahydrat versetzt und auf 70 - 80 °C erhitzt. Nach Zusatz von 320 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 10,3 g Natriumhydroxid (als 50 %ige Lösung) benötigt. Nach ca. 8 h Reaktionszeit war die Alkylierung beendet. Man ließ die Betain-Mischung auf 50 °C abkühlen und stellte den pH-Wert mit 5,4 g Citronensäure auf 5,8 ein.

Das Endprodukt war eine klare Flüssigkeit mit einer Viskosität von 112 mPas bei 20 °C, einem Trockenrückstand von 45,1 % und einem Chlorid-Gehalt von 5,1 %.

### Beispiel 8:

97,3 g Monochloressigsäure-Lösung wurden unter Kühlung in einem 1 1 Kolben mit Rührer, Innenthermometer und pH-Elektrode mit 82 g Natriumhydroxid-Lösung (50 %) vorsichtig neutralisiert. Nach der Neutralisation wurde die Natriumchloracetat-Mischung mit 26 g Magnesiumchlorid-Hexahydrat versetzt und auf 70 - 80 °C erhitzt. Nach Zusatz von 320 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 5,3 g Natriumhydroxid (als 50 %ige Lösung) benötigt. Nach ca. 8 h Reaktionszeit war die Alkylierung beendet. Man ließ die Betain-Mischung auf 50 °C abkühlen und stellte den pH-Wert mit 3,5 g Citronensäure auf 5,9 ein.

Das Endprodukt war eine klare Flüssigkeit mit einer Viskosität von 107 mPas bei 20 °C, einem Trockenrückstand von 44,4 % und einem Chlorid-Gehalt von 4,3 %.

### Beispiel 9:

97,3 g Monochloressigsäure wurden unter Kühlung in einem 1 l Kolben mit Rührer, Innenthermometer und pH-Elektrode mit 535 g Wasser verdünnt und mit 32 g Magnesiumhydroxid vorsichtig neutralisiert. Nach Zusatz von 24,3 g Trimethyglycin und 320 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 7,3 g Magnesiumhydroxid benötigt. Nach ca. 8 h Reaktionszeit war die Alkylierung beendet. Man ließ die Betain-Mischung auf 50 °C abkühlen und stellte den pH-Wert mit 18 g Citronensäure-Monohydrat auf 5,7 ein.

Das Endprodukt war eine klare Flüssigkeit mit einer Viskosität von 132 mPas bei 20 °C, einem Trockenrückstand von 45,4 % und einem Chlorid-Gehalt von 3,5 %.

## Patentansprüche

1. Verfahren zur Herstellung hochkonzentrierter fließ- und pumpfähiger wäßriger Lösungen von Betainen der allgemeinen Formel (III)
R³-CONH-(CH₂)ₘ-N⁺R⁴R⁵(CH₂)_{y}COO⁻ (III)
worin R³ der Alkylrest einer Fettsäure, welcher gegebenenfalls verzweigt, gegebenenfalls Mehrfachbindungen, gegebenenfalls Hydroxylgruppen enthalten kann, R⁴ und R⁵ gleich oder verschiedenen Alkylreste mit 1 - 4 C-Atomen bedeuten und m = 1 - 3 und y = 1, 2, 3 sein kann, durch Quaternierung von tertiären Aminstickstoff enthaltenden Verbindungen mit ω-Halogencarbonsäuren nach bekannten Verfahren, dadurch gekennzeichnet, daß der Reaktionsmischung vor oder während der Quaternierungsreaktion a) 30 - 100 Equivalent-%, bezogen auf eingesetzte ω-Halogencarbonsäure, Mg(OH)₂ und/oder Ca(OH)₂ und b) 0 - 70 Equivalent-%, bezogen auf eingesetzte ω-Halogencarbonsäure, NaOH, KOH, NH₄OH, zugesetzt werden.

2. Verfahren gemäß Anspruch 1, dadurch gekennzeichnet, daß als ω-Halogencarbonsäure Chloressigsäure eingesetzt wird.

3. Abänderung des Verfahrens nach Anspruch 1, dadurch gekennzeichnet, daß als Komponente a) 30 - 100 Equivalent-%, bezogen auf eingesetzte ω-Halogencarbonsäure, LiOH eingesetzt werden.

4. Betainlösungen, hergestellt nach einem der Ansprüche 1 bis 3, dadurch gekennzeichnet, daß die in der Lösung enthaltenen Salze zu 30 - 100 Equivalent-% Lithium-, Magnesium- und/oder Calciumsalze sind.

5. Betainlösungen, hergestellt gemäß den Ansprüchen 1 bis 3, dadurch gekennzeichnet, daß sie enthalten
| | |
|---|---|
| >35 Gew.-% | Verbindungen der allgemeinen Formel (III) |
| 4 - 8 Gew.-% | mindestens eines Lithium-, Magnesium-, Calciumsalzes |
| ad 100 | Wasser. |

6. Verfahren zur Herstellung hochkonzentrierter fließ- und pumpfähiger wäßriger Lösungen von Betainen durch Quaternierung von tertiären Aminstickstoff enthaltenden Verbindungen mit ω-Halogencarbonsäuren nach bekannten Verfahren, dadurch gekennzeichnet, daß man dem Fettsäureamid der allgemeinen Formel I und dem Na-, K- oder NH₄-Salz der ω-Halogencarbonsäure vor oder während der Quaternierungsreaktion 0,5 - 5 Gew.-%, bezogen auf Gesamtmischung, mindestens eine der Verbindungen
(Li¹⁺)ₐ(Xⁿ⁻)_{b}, (Mg²⁺)ₐ(Xⁿ⁻)_{b}, (Ca²⁺)ₐ(Xⁿ⁻)_{b}
zusetzt, worin Xⁿ⁻ der Rest einer organischen oder anorganischen Säure mit n = 1 - 3 und im Molekül der Wert von a⁺ = b·n⁻ ist.

7. Verfahren gemäß Anspruch 6, dadurch gekennzeichnet, daß die Li⁺, Mg²⁺-, Ca²⁺-Salze teilweise ersetzt werden durch Verbindungen der allgemeinen Formel (IV), 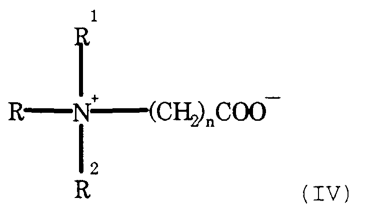 worin R, R¹, R² gleich oder verschieden gegebenenfalls verzweigter, gegebenenenfalls Hydroxylgruppen enthaltende Alkylreste mit 1 - 10 C-Atomen, insbesondere Methylreste und n = 1 - 3 ist.

8. Verfahren gemäß Anspruch 6, dadurch gekennzeichnet, daß Xⁿ⁻ der Rest einer einwertigen (n = 1) organischen oder anorganischen Säure ist.

9. Verfahren gemäß Anspruch 6, dadurch gekennzeichnet, daß Xⁿ⁻ das Anion Cl⁻ ist.

10. Betainlösungen, hergestellt gemäß den Ansprüchen 6 bis 9, dadurch gekennzeichnet, daß sie enthalten
>35 Gew.-% Verbindungen der allgemeinen Formel (III)
0,5 - 5 Gew.-% mindestens eines Lithium-, Magnesium-, Calciumsalzes.

## Claims

1. Process for the preparation of highly concentrated, free-flowing and pumpable, aqueous solutions of betaines of the general formula (III)
R³-CONH-(CH₂)ₘ-N⁺R⁴R⁵(CH₂)_{y}COO⁻ (III),
wherein R³ is the alkyl radical of a fatty acid, which radical is optionally branched, may optionally contain multiple bonds and may optionally contain hydroxyl groups, R⁴ and R⁵ are identical or different alkyl radicals having from 1 to 4 carbon atoms and m may be from 1 to 3 and y may be 1, 2 or 3, by quaternisation of compounds containing tertiary amine nitrogen with ω-halocarboxylic acids by known processes, characterised in that a) from 30 to 100 equivalent-%, based on the ω-halocarboxylic acid used, of Mg(OH)₂ and/or Ca(OH)₂ and b) from 0 to 70 equivalent-%, based on the ω-halocarboxylic acid used, of NaOH, KOH or NH₄OH are added to the reaction mixture before or during the quaternisation reaction.

2. Process according to claim 1, characterised in that chloroacetic acid is used as the ω-halocarboxylic acid.

3. Modification of the process according to claim 1, characterised in that there is used as component a) from 30 to 100 equivalent-%, based on the ω-halocarboxylic acid used, of LiOH.

4. Betaine solutions, prepared according to any one of claims 1 to 3, characterised in that the from 30 to 100 equivalent-% of salts contained in the solution are lithium salts, magnesium salts and/or calcium salts.

5. Betaine solutions, prepared according to claims 1 to 3, characterised in that they contain
| | |
|---|---|
| >35% by weight | of compounds of the general formula (III) |
| from 4 to 8% by weight | of at least one lithium, magnesium or calcium salt |
| *ad* 100 | water. |

6. Process for the preparation of highly concentrated, free-flowing and pumpable, aqueous solutions of betaines by quaternisation of compounds containing tertiary amine nitrogen using ω-halocarboxylic acids according to known processes, characterised in that from 0.5 to 5% by weight, based on the total mixture, of at least one of the compounds
(Li¹⁺)ₐ(Xⁿ⁻)_{b}, (Mg²⁺)ₐ(Xⁿ⁻)_{b} and (Ca²⁺)ₐ(Xⁿ⁻)_{b},
wherein Xⁿ⁻ is the radical of an organic or inorganic acid and n is from 1 to 3 and in the molecule the value of a⁺ is b · n⁻, is added to the fatty acid amide of the general formula (I) and the Na, K or NH₄ salt of the ω-halocarboxylic acid before or during the quaternisation reaction.

7. Process according to claim 6, characterised in that the Li⁺, Mg²⁺, Ca²⁺ salts are partially replaced by compounds of the general formula (IV) 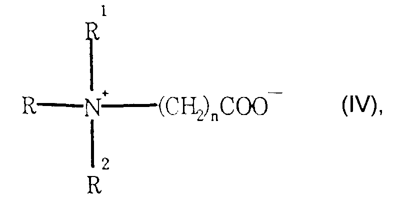 wherein R, R¹ and R² are identical or different, optionally branched, optionally hydroxyl group-containing alkyl radicals having from 1 to 10 carbon atoms, especially methyl radicals, and n is from 1 to 3.

8. Process according to claim 6, characterised in that Xⁿ⁻ is the radical of a monobasic (n = 1 ) organic or inorganic acid.

9. Process according to claim 6, characterised in that Xⁿ⁻ is the Cl⁻ anion.

10. Betaine solutions prepared according to claims 6 to 9, characterised in that they contain
>35% by weight of compounds of the general formula (III)
from 0.5 to 5% by weight of at least one lithium, magnesium, calcium salt.

## Revendications

1. Procédé pour préparer des solutions aqueuses de bétaïnes, fluides et pompables, très concentrées, de formule générale (III)
R³-CONH-(CH₂)ₘ-N⁺R⁴R⁵(CH₂)_{y}COO⁻ (III)
dans laquelle R³ est le résidu alkyle d'un acide gras, qui est éventuellement ramifié et peut contenir éventuellement des liaisons multiples et éventuellement des groupes hydroxyle, R⁴ et R⁵ sont des groupes alkyle identiques ou différents ayant de 1 à 4 atomes de carbone et m peut valoir de 1 à 3 et y peut prendre les valeurs 1, 2 et 3, par quaternisation de composés contenant un azote d'amine tertiaire avec des acides ω-halogénocarboxyliques par des procédés connus, caractérisé en ce que le mélange réactionnel est, avant ou pendant la réaction de quaternisation, additionné a) de 30 à 100 % en équivalents, par rapport à l'acide ω-halogénocarboxylique utilisé, de Mg(OH)₂, et b) de 0 à 70 % en équivalents, par rapport à l'acide ω-halogénocarboxylique utilisé, de NaOH, de KOH ou de NH₄OH.

2. Procédé selon la revendication 1, caractérisé en ce que, en tant qu'acide ω-halogénocarboxylique, on utilise de l'acide chloracétique.

3. Variante du procédé selon la revendication 1, caractérisée en ce que, en tant que constituant a), on utilise de 30 à 100 % en équivalents de LiOH par rapport à l'acide ω-halogénocarboxylique utilisé.

4. Solutions de bétaïne, préparées selon l'une des revendications 1 à 3, caractérisées en ce que les sels contenus dans la solution sont, pour 30 à 100 % en équivalents, des sels de lithium, de magnésium et/ou de calcium.

5. Solutions de bétaïne préparées selon les revendications 1 à 3, caractérisées en ce qu'elles contiennent plus de 35 % en poids de composés de formule générale (III), 4 à 8 % en poids d'au moins un sel de lithium, de magnésium, de calcium, le complément à 100 étant constitué d'eau.

6. Procédé pour préparer des solutions aqueuses de bétaïnes, fluides et pompables, à forte concentration, par quaternisation de composés contenant un azote d'amine tertiaire avec des acides ω-halogénocarboxyliques par des procédés connus, caractérisé en ce qu'on ajoute à l'amide d'acide gras de formule générale (I) et au sel de Na, de K ou de NH₄ de l'acide ω-halogénocarboxylique, avant ou pendant la réaction de quaternisation, de 0,5 à 5 % en poids, par rapport au mélange total, d'au moins l'un des composés
(Li¹⁺)ₐ(Xⁿ⁻)_{b}, (Mg²⁺)ₐ(Xⁿ⁻)_{b}, (Ca²⁺)ₐ(Xⁿ⁻)_{b}
où Xⁿ⁻ est le résidu d'un acide organique ou minéral dans lequel n vaut 1 à 3, et, dans la molécule, a⁺ = b.n⁻.

7. Procédé selon la revendication 6, caractérisé en ce que les sels de Li⁺, Mg²⁺, Ca²⁺, sont en partie remplacés par des composés de formule générale (IV) 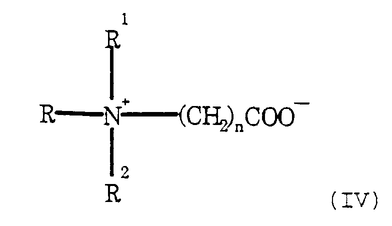 dans laquelle R, R¹, R² sont des groupes alkyle ayant de 1 à 10 atomes de carbone, identiques ou différents, éventuellement ramifiés, contenant éventuellement des groupes hydroxyle, en particulier des groupes méthyle, et n vaut de 1 à 3.

8. Procédé selon la revendication 6, caractérisé en ce que Xⁿ⁻ est le résidu d'un monoacide (n = 1) organique ou minéral.

9. Procédé selon la revendication 6, caractérisé en ce que Xⁿ⁻ est l'anion Cl⁻.

10. Solutions de bétaïne préparées selon les revendications 6 à 9, caractérisées en ce qu'elles contiennent > 35 % en poids de composés de formule générale (III) et de 0,5 à 5 % en poids d'au moins un sel de lithium, de magnésium, de calcium.
